(19) Europäisches Patentamt
European Patent Office
Office européen des brevets

(11) Publication number : **0 559 603 A2**

(12) **EUROPEAN PATENT APPLICATION**

(21) Application number : **93810003.9**

(22) Date of filing : **05.01.93**

(51) Int. Cl.[5] : **C12N 15/82,** C12N 15/29, C12N 5/10, A01H 5/00

The applicant has subsequently filed a sequence listing and declared, that it includes no new matter.

(30) Priority : **09.01.92 US 791929**

(43) Date of publication of application : **08.09.93 Bulletin 93/36**

(84) Designated Contracting States : **AT BE CH DE DK ES FR GB GR IE IT LI LU NL PT SE**

(71) Applicant : **SANDOZ LTD.**
**Lichtstrasse 35**
**CH-4002 Basel (CH)**
(84) **BE CH DK ES FR GB GR IE IT LI LU NL PT SE**

(71) Applicant : **SANDOZ-PATENT-GMBH**
**Humboldtstrasse 3**
**D-79539 Lörrach (DE)**
(84) **DE**

(71) Applicant : **SANDOZ-ERFINDUNGEN Verwaltungsgesellschaft m.b.H.**
**Brunner Strasse 59**
**A-1230 Wien (AT)**
(84) **AT**

(72) Inventor : **Brunke, Karen J.**
**11 Somerset Court**
**Belmont, CA 94002 (US)**
Inventor : **Wilson, Stacey L.**
**19387 Greenwood Drive 3**
**Cupertino, CA 95014 (US)**

(54) **Brassica hsp 80 promoter.**

(57) A Brassica promoter (hsp80) has been isolated which can provide for constitutive expression of heterologous genes in a wide range of tissues and organs. Various deletion mutants and hybrid promoters are described which retain activity and/or show enhanced activity. Upstream activating sequences are described which separately and in combination can provide for constitutive gene expression. These sequences can also confer constitutive expression on heterologous, non-constitutive promoters.



Jouve, 18, rue Saint-Denis, 75001 PARIS

This invention relates to a novel promoter which is functional in plants, more specifically to a promoter which controls expression of a desired gene in a constitutive manner. It also includes various upstream sequences which can be used to construct hybrid promoters having desired activity.

## BACKGROUND OF THE INVENTION

Heat shock genes (hsp genes) are known in a variety of organisms, including yeast, Drosophila, and some plant species. One class of heat shock genes expresses protein in response to heat stress only. Another class of heat shock genes has a low basal level of activity that is highly elevated upon heat induction.

When genetically engineering a heterologous gene in plants, the selection of a promoter is often a critical factor. While it may be desirable to express certain genes only in response to a particular stimulus, or localise their expression in certain tissues, other genes are more desirably expressed constitutively, i.e., throughout the plant at all times and in most tissues. In the past, the 35S promoter from Cauliflower Mosaic Virus (CaMV) has been used for constitutive expression of heterologous genes. For regulatory and other reasons it would be desirable to regulate heterologous gene expression with a promoter which is not of pathogenic origin. In addition, use of a plant promoter may alter the level of activity in particular tissues and may alter the spectrum of tissues in which expression is achieved in comparison with viral promoters.

## DESCRIPTION OF THE INVENTION

This invention relates to a constitutive promoter from cauliflower (Brassica oleracea cv. 'Delira'), i.e. one which will express genes at all times and in most tissues and organs. The promoter may be operatively linked to any desired gene and it will direct the expression of that gene. This promoter can be distinguished from promoters of previously described hsp genes in that it has a high basal level of constitutive expression and little increased expression upon heat induction.

This promoter has been designated the "hsp80 promoter" since it is a constitutive promoter with a heat shock consensus element and is taken from a gene which has some homology to the hsp80 family of genes from other species. The hsp80 promoter directs the production of heat shock proteins at a high basal level at normal temperatures (20-25°C) and shows slightly elevated expression with heat stress (35-40°C).

## DESCRIPTION OF THE FIGURES

Figure 1 shows plasmid pZ0217 and the construction of plasmids pZ0601 and pZ0601BS.

Figure 2 is a representation of pZ0602.

The sequence of the complete promoter is given in TABLE 1. (SEQ.ID. NO: 1). The complete native sequence has 1568 base pairs. The nucleotides are numbered in negative order from the translation start site (ATG) for the native heat shock protein. The following areas have been identified in this sequence:

A) A "TATA" box is an eight base pair sequence "TATATATA" located from -97 to -90, inclusive.

B) There is a 61 bp mRNA leader sequence which is located from -61 to -1.

C) A cap site has been identified at -61.

D) There is a region from -604 to -488 which appears to contain an upstream activating sequence for this promoter. This area is designated UAS 1. This area had been observed to confer constitutive activity in a transient assay and had previously been designated "constitutive box".

E) There is a region from -1000 to -604 which appears to contain a further upstream activating sequence. This area is designated UAS 2.

F) There is a region from -488 to -120 which appears to contain a further upstream activating sequence. This area is designated UAS 3.

G) Two direct repeats exist, between -779 to -741 and between -740 to -702. Also, a sequence containing a portion of the direct repeat extends from -701 to -677.

H) One heat shock consensus element is located at -131 to -120 and another element is located at -244 to -237.

Thus one aspect of this invention provides a DNA construct comprising a Brassica hsp80 promoter operably linked to a heterologous gene. Since it is appreciated that minor changes may be made in this DNA sequence without substantially affecting the promoter's activity, this invention also includes DNA sequences which are the "functional equivalent" of a Brassica hsp80 promoter and constructs comprising such DNA sequences operably linked to a heterologous gene.

As used throughout the specification and claims, the following definitions apply:

"Functional equivalent" is any DNA sequence which is complementary to a DNA sequence which, under stringent hybridisation conditions, will hybridise with the reference sequence and has promoter activity similar to the Brassica hsp 80 promoter.

"Stringent hybridisation conditions" are those in which hybridisation is effected at 60°C in 2.5X saline citrate buffer (SSC buffer) followed by merely rinsing at 37°C at a reduced buffer concentration which will not affect the hybridisations which take place.

"Heterologous gene" is a DNA sequence coding for any peptide or protein other than the Brassica hsp80 protein.

"Deletion promoter" is any Brassica hsp80 promoter which has a deletion and still retains activity.

"Functional equivalent of a deletion promoter" is a deletion promoter which has had further deletions, yet retains at least substantially equivalent activity as compared with the deletion promoter.

"Regulatable promoter" is any promoter whose activity is affected by cis or trans acting factor(s).

"Constitutive promoter" is any promoter which is active in most tissues or organs at most times.

The hsp80 promoter (or its functional equivalent) may be used to constitutively express any heterologous gene desired. Examples of suitable heterologous genes, include, without limitation: insecticidal toxins (such as those from Bacillus thuringiensis), herbicide resistance genes, antimicrobial genes, anti-fungal genes, anti-viral genes, and anti-feedant genes.

It is preferred that the hsp80-heterologous gene construct be inserted into a vector, and that vector be used to transform a eukaryotic host. The eukaryotic host is preferably a plant cell or a plant protoplast. Preferred vectors will, of course vary depending on the chosen host. For dicotyledons, the vector may be introduced into a protoplast by electroporation or the vector may be an Agrobacterium tumefaciens (A.t.) Ti-plasmid derivative which infects the cell or protoplast and may be employed in A.t. mediated transformation including so-called binary techniques. (See, e.g. Gasser C.S. et al. 1989, Science 244:1293- 1299). Monocotyledons are preferably transformed using the so-called" ballistic" technique (Gasser et al, supra) or may also be transformed using protoplasts.

In either case, appropriate transformation vectors and transformation protocols are well known in the art. The transformed cells or protoplasts are cultured in an appropriate culture medium, and a transformed plant is regenerated. The transformed plant expresses the heterologous gene constitutively.

It has also been surprisingly found that various deletions may be made in this promoter and the resulting deletion promoter is found to have either: a) enhanced activity; b) substantially the same activity as the native promoter; or c) retained activity.

Thus another aspect of this invention is a DNA sequence comprising a deletion promoter of a Brassica hsp 80 promoter. A further aspect of this invention is a DNA construct comprising a deletion promoter operably linked to a heterologous gene. Also included in this aspect of the invention are functional equivalents of deletion promoters and constructs comprising a functionally equivalent deletion promoter and a heterologous gene.

Various deletion and hybrid promoters were made as detailed in the Examples. A first set of deletion promoters is designated the 601BS series. These promoters are characterised by having a deletion which includes the base pairs from -118 to -246. It was found that one promoter from this series, 601BSΔ 2-3, which contains a deletion from -493 to -118 retains approximately 50%-75% activity compared to the intact promoter.

The second set of deletion promoters is designated the 602 series. These promoters all have a deletion from at least from -488 to -134, and may have a 5' end deletion of varying length, as summarised in Example 3. Surprisingly, some deletions enhance activity.

Deletion promoter 603 has a deletion spanning from -1125 to -134. This promoter retained only about 10% of the activity of the intact promoter. Deletion promoter 604 has deletions from -1568 to -1125 and from -496 to -134, retaining approximately 25% activity. Deletion promoter 605 has deletions of all base pairs upstream of -488, and a corresponding decrease in activity to only about 6-8%.

One particularly important area for activity lies between -134 and -120. Deletion promoter 601BS(BSph) only has this small area deleted, but its activity dropped to only about 50-75% of the intact promoter. Preferred promoters of this invention therefore contain at least this short sequence.

As mentioned supra, the 116 bp region ranging from -604 to -488 (UAS 1) or part thereof appears to be responsible for conferring constitutive activity in a range of tissues. UAS 2 and UAS 3 as described above appear to confer activity in further tissues. Therefore, another aspect of this invention is conferring constitutive activity on an otherwise non-constitutive promoter (such as one which is normally inducible or otherwise regulatable) by operably linking to or inserting within an inducible or regulatable promoter one or more upstream activating regions which alone produce activity in some to most organs/tissues and combined give so-called constitutive activity. The invention includes DNA constructs comprising such promoters with conferred constitutive activity operatively linked to a structural gene, processes e.g. for the transformation of plant cells and protoplasts using the constructs and plant cells and protoplasts transformed with the constructs.

It is recognised that it is possible that regions smaller than the UAS 1, 2 and 3 regions will be sufficient to confer constitutive activity. This can be tested by making deletions in these regions using methods which are well known in the art. The promoters with deletions in the UAS regions can then be assayed for retention of constitutive activity. Such assays are also within the skill of the ordinary artisan.

The UAS 1, 2 and 3 regions alone or together can also be used to restore activity to a promoter which has been rendered inactive, by deletions and/or mutation. This also forms another aspect of the invention. One example of this use would be with the CaMV 35S promoter which has been deleted until it is no longer functional. Insertion of the UAS 1, 2 and 3 elements in combination or alone will restore promoter activity in some or all tissues.

The invention is further illustrated in the following non-limiting Examples.

**EXAMPLE 1 - hsp80 Promoter Isolation**

A genomic library of Brassica oleracea (cv. 'Delira') is constructed in Charon 35 Lambda phage and K802 cells using the methods essentially as described by Maniatis, et al. 1982 Molecular Cloning, Cold Spring Harbor Laboratory, p. 282-283, which is hereby incorporated by reference. This library is screened with a PvuI-StuI fragment from the Drosophila hsp83 gene [Hackett, R.W. et al. 1983 Nucl.Acids Res. 11(20):7011-7030]. Twenty recombinants with apparent homology to the Drosophila gene are recovered, and Southern blot analysis is performed using the Drosphila hsp83 gene fragment as a probe. A 5.8 kb HindIII fragment is chosen for subcloning in a pUC9 vector, and is referred to as pZ0217. This plasmid is illustrated in Figure 1.

Next, a Chloramphenicol acetyl transferase (CAT) gene (Pharmacia) is inserted into the PstI site of the known vector pUC19. Then the NOS terminator [Bevan, M. eg al. 1983. "Structure and Transcription of the Nopaline Synthase Gene Region of T-DNA" Nucl. Acids Res. 11(2)] is inserted at the PstI-HindIII site. This resulting plasmid is designated pZ030. The BglII fragment from pZ0217 is separated and subcloned into the BamHI site of pZ030. This results in a promoter-CAT gene-NOS terminator construct which is transferred as an EcoRI-HindIII fragment to the commercially available vector pTZ18R (from Pharmacia), creating pZ0601, as illustrated in Fig. 1.

**EXAMPLE 2 - Plasmid Constructions**

In vitro mutagenesis of the hsp80 promoter in pZ0601 is accomplished using the Oligonucleotide Directed In Vitro Mutagenesis System supplied by Amersham. Two oligonucleotides are synthesised according to manufacturer's instructions to create two unique restriction sites within the promoter upstream of the TATA box, BamHI at -134 and SphI at -120. (All nucleotide positions are given relative to the translation initiation site.) This new plasmid is designated pZ0601BS, and is also shown in Figure 1.

**Plasmid pZ0602 and pZ0603**

Step A) pZ0601BS is digested with BamHI and the ends are filled in using the Klenow fragment of DNA polymerase. The DNA is then digested with KpnI and the resulting promoterless fragment is separated on a low-melting point agarose gel.

Step B) pZ0601BS is digested with BamHI and KpnI and the hsp80 promoter is separated on a low-melting point agarose gel. This fragment is purified using an ELUTIP (Schleicher and Schnell) and is digested with either DraI or FnuDII.

Step C) A -1568 to -488 DraI fragment from step B) is ligated into the promoterless fragment of Step A. The resulting plasmid is designated pZ0602, and is shown in Figure 2.

Step D) A -1568 to -1125 FnuDII fragment is ligated into the promoterless fragment from Step A. The resulting plasmid is designated pZ0603.

**Plasmid pZ0604**

Step A) pZ0601BS is digested with BamHI and SmaI and the BamHI site is filled in with T4 DNA polymerase and deoxynucleotides. The resulting promoterless fragment is separated on a low-melting point agarose gel.

Step B) pZ0601BS is digested with FnuDII. The -1125 to -496 FnuDII fragment is ligated into the promoterless fragment of Step A to result in pZ0604.

**Plasmid pZ0605**

Step A) pZ0601BS is digested with BamHI and SmaI and the resulting promoterless fragment is separated on a low-melting point agarose gel.

Step B) pZ0601BS is digested with DraI. A -488 to -134 DraI fragment is ligated into the promoterless fragment of Step A to construct pZ0605.

**Deletion Mutants**

A series of 5' deletions in the hsp80 promoter is constructed from the pZ0602 plasmid. pZ0602 is digested with SacI and SmaI to create a substrate for Exonuclease III (Stratagene) digestion. After treatment with Exonuclease III for varying lengths of time, the resulting DNAs are blunted with Mung Bean Nuclease (Boehringer Mannheim). The DNAs are separated using low-melting point agarose gel electrophoresis. The prominent bands are excised, diluted and ligated. After transformation, deletion mutants are chosen and sequenced through the junction point.

A series of 3' deletions in the hsp80 promoter is constructed from pZ0601BS by digesting with BamHI and SphI and then following the same procedure as described to create the 5' deletions.

**EXAMPLE 3 - Bioassays**

**Carrot cell line maintenance**

Redwood City Wild Carrot (RCWC) suspension culture (obtained from Stanford University) is maintained in the following Carrot Suspension Medium: 1 X MS salts, 1 mg/l nicotinic acid, 1 mg/l pyridoxine HCl, 1 mg/l thiamine, 100 mg/l inositol, 0.1 mg/l 2,4-D, 30 g/l sucrose, and adjusted to a final pH of 5.8 with KOH. The culture is maintained by diluting 1:10 into fresh medium every 7 days.

**Protoplast Formation**

RCWC suspension culture is diluted 1:10 four days prior to use. 50 ml culture (approximately 5 mls packed cell volume) is centrifuged for 10 minutes at 500 g. The cells are then resuspended in 50 mls of the following filtered Carrot Enzyme Solution: 10 g/l Cellulysin (Calbiochem), 5 g/l Rhozyme (Genecor), 0.4 M mannitol, 50 mM CaCl2, 10 mM NaOAc, pH 5.8. Cells are rocked gently for two hours to digest. Protoplasts are washed twice and resuspended in Carrot Culture Medium (CCM), which is the same as Carrot Suspension Medium described above with the addition of 0.4 M mannitol. Protoplasts are counted on a hemacytometer at a 1:10 dilution to determine concentration.

**Electroporation**

A PG200 Progenitor II (Hoefer) with a circular electrode is used for all electroporations. Samples are electroporated in 24-well sterile microtitre dishes at 250 volts for 100 msec.

Each CAT (Pharmacia) construct-containing plasmid is tested in 3 or 4 replicates in multiple experiments. 30 to 50 μg of plasmid pZ0601BS is used in each experiment as a control. All other plasmids are tested using an equivalent molar amount of DNA as compared to pZ0601BS.

Approximately $10^6$ protoplasts are aliquoted into 1.5 ml tubes, centrifuged for two minutes at 500 g, and most of the supernatant is removed. Each DNA is added to 75 μl 2M KCl. The volume is adjusted to 1 ml with the addition of CCM (pH adjusted to 8.0) and this mixture is electroporated and immediately diluted into 5 mls CCM pH 5.8 in a petri dish. Diluted samples are stored in a dark cupboard for 1-2 days before collection for CAT assays.

| Promoter | Regions deleted | Relative Activity |
|---|---|---|
| 601BS | None | 100% |
| 602 Δ3-2 | -1568 to -1000 and -488 to -134 | 125-175% |
| 602 Δ3-3 | -1568 to -948 and -488 to -134 | 75-125% |
| 602 Δ4-9 | -1548 to -830 and -488 to -134 | 100-150% |
| 602 Δ4-6 | -1548 to -628 and -488 to -134 | 75-100% |
| 601BS Δ2-3 | -493 to -118 | 50- 75% |
| 603 | -1125 to -134 | Approx.10% |
| 604 | -1568 to -1125 and -496 to -134 | Approx. 25% |
| 605 | -1568 to -488 | 6- 8% |
| 601BS(BSph) | -134 to -120 | 50- 75% |

**EXAMPLE 4 - Constitutive Expression in Complete Plant**

Tobacco is transformed using the following protocol.

**Plant Tissue**

Tobacco leaf explants are obtained from sterilely-grown tobacco plants. The sterile tobacco plants are vegetatively propagated at approximately 1 month intervals by removing the top nodes of the existing plant and reculturing them in a sponge jar containing 75 ml of agar-solidified hormone-free medium (0/0 medium) containing Murashige-Skoog salts, 1 ml/l of 100 mg/l myo-inositol, 5 ml/l vitamix (which provides 0.5 mg/l pyridoxine HCl, 0.5 mg/l nicotinic acid, and 1.0 mg/l thiamine) and 3% sucrose. Tobacco plants are maintained at medium intensity continuous light. Jars are sealed with green paper tape, or may be left unsealed to allow gas exchange.

**Agrobacterium vector**

A binary vector system is used to transform Agrobacterium tumefaciens, and the bacteria are then used to transform tobacco cells.

**GENERAL PROCEDURES**

Agrobacterium vectors are stored at -70°C. Approximately 18 hours before transformation, 25 ml of sterilised liquid LB3 medium (below) containing the appropriate antibiotics is inoculated with 100-500 microliters Agrobacterium culture. The culture is grown on a 28°C shaker at 250 rpm overnight.

| LB3 Medium (for 1 liter) | |
|---|---|
| Tryptone | 10 g |
| Yeast Extract | 5 g |
| NaCl | 4 g |
| KCl | 1 g |
| $MgSO_47H_2O$ | 3 g |

Add $H_2O$ to 1 litre, and dispense in 25 ml portions per flask.

Antibiotics are 25 μg/ml streptomycin and 50 μg/ml kanamycin. Transformation is generally performed when the cells are in their log phase; O.D. at 600 nm should preferably be 0.50-1.0. The culture should be diluted to a concentration of 108 cells/ml in 50 ml liquid hormone-free 0/0 medium.

Tobacco leaf explants are dipped for 3 minutes in $10^8$ cells/ml dilution of transformed Agrobacterium as prepared above. Explants are removed and blotted dry on sterile Whatman filter paper. They are then placed on regeneration medium with no antibiotics for two days. On day 3, the treated explants are transferred to regeneration medium containing appropriate antibiotics. Explants remain on these plates for 3 to 4 weeks or until putatively transformed shoots are large enough to move to the rooting medium. The rooting medium is half-strength 0/0 medium containing 250 mg/l carbenicillin and either 100 mg/l kanamycin or 50 mg/l hygromycin, depending on the construct. After approximately two weeks shoots will be rooted and green. They are then transferred into jars and each plant is given its own identification number. Assays are then performed on various tissues to demonstrate constitutive promoter activity.

## TRANSFORMATION VECTORS

### pZ0639

A plasmid is constructed similarly to pZ0601 except instead of containing the hsp80-CAT gene-NOS terminator, it contains a hsp-80-GUS-NOS terminator cassette. This plasmid is designated pZ0612.

Plasmid pBin19 (Clonetech) is cut with EcoRI and HindIII. Plasmid pZ0612 is cut with ScaI, EcoRI and HindIII. Both digests are separated on low melt agarose. A 12 kb-pBin19 vector fragment and a ~3.6 Kb hsp80-GUS-NOS fragment are isolated and subsequently ligated together. The resulting plasmid, designated pZ0639 can be identified with a PstI digest.

### pZ0640

Following the procedure described supra, pZ0640 is constructed. It differs from pZ0639 by having a truncated hsp80 fragment (.624 kb vs the full 1.56 promoter).

### pZ0641

Following the procedure described supra, pZ0641 is constructed. pZ0641 contains a .252 kb hsp80 promoter rather than a full length promoter.

### pZ0642

Following procedures described supra, pZ0642 is constructed. Rather than an hsp80 or derivative promoter, pZ0642 contains the CaMV 35S promoter-GUS-NOS fragment.

## EXAMPLE 5 - Promoter activity

Plants obtained from the procedure of Example 4 are tested for expression of the heterologous gene, GUS. Tissue samples are taken and assayed for the presence of GUS. GUS activity is detected in all tissue samples. Control tissues (obtained from non-transformed plants which were subjected to the same culture and regeneration procedure) are also assayed for GUS activity; none is measured. The number in parenthesis is total number of plants sampled. Results are given as the number of positives (blue color is observed). "NT" is not tested; "NA" is not available; "M" is a mutant.

| Plasmid | Construct (plus GUS; NOS terminator) |
|---|---|
| pZ0639 | Full length hsp80 |
| pZ0640 | -1000 to -488 + -134 to -23 of hsp80 (comprises UAS1 + UAS2 + TATAA region) |
| pZ0641 | -628 to -488 + -134 to -23 of hsp80 (comprises UAS1 +TATA region) |
| pZ0642 | 35S promoter |

| Tissue (7) | pZ0639 (7) | pZ0640 (6) | pZ0641 (5) | pZ0642(7) |
|---|---|---|---|---|
| leaf mesophyll | 1/7 | 1/5; 1NT | 1/5 | 5/7 |
| leaf vein | 5/7 | 1/6 | 2/5 | 3/7 |
| leaf trichomes | 4/7 | 1/6 | 2/5 | 1/7 |
| lateral meristem | 2/7 | 0/5; 1NT | 1/5 | 4/7 |
| lateral trichomes | 1/7 | 0/6 | 0/5 | 1/7 |
| sepal veins | 3/7 | 3/6 | 2/4; 1NA | 4/6; 1NA |
| sepal trichomes | 4/7 | 1/6 | 0/4 | 0/6; 1NA |
| carpel | 2/7 | 4/6 | 0/4; 1NA | 4/6; 1NA |
| floral tube/petal veins | 1/7 | 1/6 | 1/4; 1NA | 3/6, 1NT |
| floral tube/petal trichomes | 4/7 | 2/6 | 0/4 | 3/6; 1NT |
| immature anther | 4/7 | 5/6 | 2/3; 1NA; 1M | 3/5; 1NA; 1NT |
| pollen | 5/5; 2NA | 5/6 | 3/3; 1NA; 1M | 0/6; 1NA |
| roots | 6/7; 1NT | 1/4; 2NT | 0/4; 1NT | 3/6; 1NT |
| stem | 2/2; 6NT | 0/2; 4NT | 0/1; 4NT | 1/1; 6NT |

This data shows that the hsp80 promoter is active to some extent in all tissues tested, although the intensity of the staining was less than the 35S promoter in most cases. In general, leaf mesophylls do not stain until vacuum infiltration, but then a large number of cells do stain, although their appearance resembles that of trichomes rather than typical mesophyll cells.

**EXAMPLE 6 - Hybrid Promoters**

Various upstream regions of the hsp80 promoter are ligated to a non-active heterologous minimal promoter to determine if the upstream region would impart activity. The fragments indicated below are cloned upstream of a truncated CaMV 35S promoter extending from -46 relative to the CaMV transcription start site to +131 (the TATAAA box is -31 to -25), hereinafter referred to as the "-46 35S promoter". [NB the numbering used here to identify truncated CaMV35S refers to CaMV35S itself and is not the same as the numbering used elsewhere for hsp80 and fragments thereof] Plasmid pZ0625 consists of the -46 35S promoter, intron 6 from the maize ADH1S gene, the β-glucuronidase (GUS) coding region, and the NOS terminator in pT7T3 18U (available from Pharmacia). The following Brassica hsp80 fragments are ligated upstream of the -46 35S promoter fragment:

| Fragment (numbered according to TABLE 1) | Plasmid |
|---|---|
| -628 to -488 plus -134 to -120 | pZ0670 |
| -1000 to -488 plus -134 to -120 | pZ0681 |
| -1000 to -604 | pZ0682 |
| -488 to -120 | pZ0683 |
| -1548 to -488 plus -134 to -120 | pZ0689 |

The hybrid promoters contained in the above plasmids are referred to as hybrid promoters 670, 681, 682, 683 and 689 respectively.

Also tested is pZ0612 (which is the same as pZ0601 except the CAT gene is replaced with a GUS gene) and consists of a full-length hsp80 promoter controlling GUS with a NOS terminator (but no intron)in pTZ 18R.

Protoplasts are prepared from carrot as described previously, Black Mexican Sweet (BMS) maize, and tobacco. Protoplasts are electroporated with the desired plasmid, allowed to recover for a day, and then extracted and extracts are assayed for GUS activity. GUS activity is measured spectro-photometrically. Results are shown as the means, normalised to the full length 35S promoter construct, pZ0663 (35S from -366 to +131, intron 6, GUS, NOS terminator). The full length 35S promoter is described in Franck et al. Cell., Vol. 21, 285-294 (1980)

| TRANSIENT GUS ASSAY | | | |
|---|---|---|---|
| Plasmid | Tobacco | Carrot | BMS |
| pZ0663 | 1.0 | 1.0 | 1.0 |
| pZ0625 | 0.03 | 0.01 | 0.03 |
| pZ0670 | 0.16 | 0.28 | 0.02 |
| pZ0681 | 0.13 | | |
| pZ0682 | 0.24 | 0.23 | 0.08 |
| pZ0683 | 0.17 | | |
| pZ0689 | 0.16 | 0.32 | 0.03 |
| pZ0612 | 0.65 | | |

| TRANSIENT CAT ASSAY | |
|---|---|
| Plasmid | Carrot |
| pZ0602 Δ4-6 | 0.75-1.0 |
| pZ0602 Δ3-2 | 1.25-1.75 |
| pZ0605 | 0.06-0.08 |
| pZ0601 BSΔ2-3 | 0.05-0.75 |
| pZ0601 BS | 1.0 |

As can be seen from these above table, all the upstream regions give essentially the same activity when fused to the 35S -46 promoter, and there appears to be little additive effect.

## Claims

1. A DNA construct comprising a Brassica hsp80 promoter or its functional equivalent, operably linked to a heterologous gene.

2. A DNA construct comprising a deletion or hybrid promoter of a Brassica hsp80 promoter, or its functional equivalent, operably linked to a heterologous gene.

3. A DNA construct according to Claim 2 wherein the deletion promoter is selected from the group consisting of deletion promoters designated: 602, 602 Δ3-3, 602 Δ4-9, 602 Δ4-6 or a functional equivalent of said deletion promoters.

4. A DNA construct according to claim 2 wherein the hybrid promoter is selected from the group consisting of the hybrid promoters designated: 670, 681, 682, 683, 689 and functional equivalents of said hybrid promoters.

5. A method of conferring constitutive activity to an inducible, otherwise regulatable, or inactivated promoter comprising:
   a) isolating one or more upstream activating regions from a Brassica hsp80 promoter; and
   b) operably linking or inserting said region or regions to or within the inducible, otherwise regulatable or inactivated promoter to obtain constitutive activity.

6. A method according to Claim 5 wherein the upstream activating region comprises the -604 to -488 region of a Brassica hsp80 promoter or part thereof.

7. A method according to Claim 5 wherein the upstream activating sequence comprises the -1000 to -604 region of a Brassica hsp80 promoter or part thereof.

8. A method according to Claim 5 wherein the upstream activating sequence comprises the -488 to -120 region of a Brassica hsp80 promoter or part thereof.

9. A constitutive promoter comprising an inducible, otherwise regulatable or inactivated promoter which is other than a Brassica hsp80 promoter and one or more upstream activating regions from a Brassica hsp 80 promoter operably linked to or inserted within the inducible, otherwise regulatable or inactivated promoter.

10. A promoter according to Claim 9 wherein the upstream activating region comprises the -604 to -488 region of a Brassica hsp80 promoter or part thereof.

11. A promoter according to claim 9 wherein the upstream activating region comprises the -1000 to -604 region of a Brassica hsp80 promoter or part thereof.

12. A promoter according to claim 9 wherein the upstream activator region comprises the -488 to -120 region of a Brassica hsp80 promoter or part thereof.

13. A DNA construct comprising a promoter according to Claim 9 operably linked to a structural gene.

14. A DNA construct according to Claim 1, 2 or 13 wherein said heterologous gene is selected from the group consisting of insecticidal genes, herbicidal resistance genes, anti-microbial genes, anti-fungal genes, anti-viral genes, and anti-feedant genes.

15. A plant cell or protoplast transformed with the DNA construct of Claim 1, 2, 13 or 14.

16. A cell or protoplast according to Claim 15 which is a dicotyledon.

17. A cell or protoplast according to Claim 15 which is a monocotyledon.

18. A DNA sequence which is a functional equivalent of a Brassica hsp 80 promoter.

19. A DNA sequence substantially as shown in Table 1 or functional equivalent thereof which hybridises under stringent conditions with the sequence shown in Table 1 and has promoter activity similar thereto, and functional equivalent parts thereof.

**20.** A DNA as shown in Table 1 and functional equivalents thereof.

**21.** A DNA sequence selected from the group consisting of UAS 1, UAS 2, UAS 3 and functional equivalent parts thereof.

**22.** A DNA sequence which is a deletion or hybrid promoter of a Brassica hsp 80 promoter.

**23.** A plant the genome of which comprises a DNA construct according to Claim 1, 2, 13 or 14.

FIG. 1.1

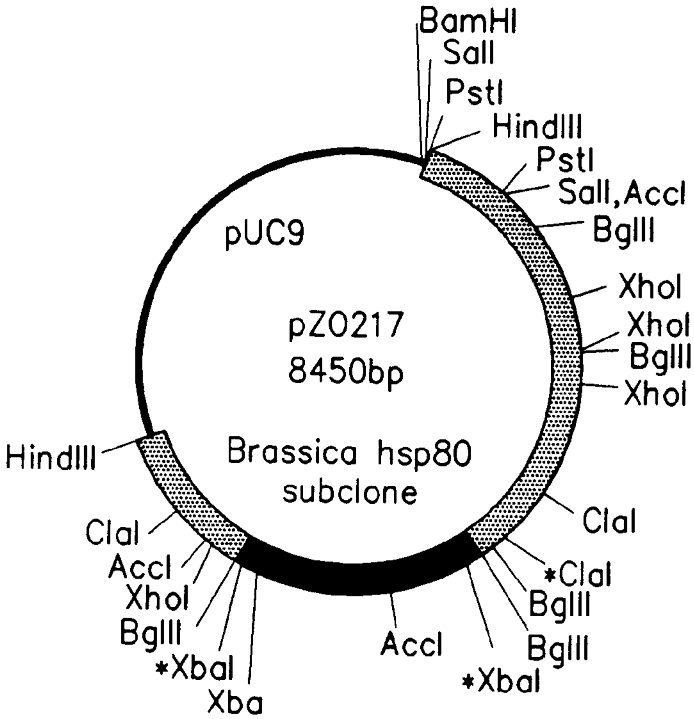
BamHI
SalI
PstI
HindIII
PstI
SalI,AccI
BglII
XhoI
XhoI
BglII
XhoI
ClaI
*ClaI
BglII
BglII
*XbaI
AccI
Xba
*XbaI
BglII
XhoI
AccI
ClaI
HindIII
pUC9
pZO217
8450bp
Brassica hsp80
subclone

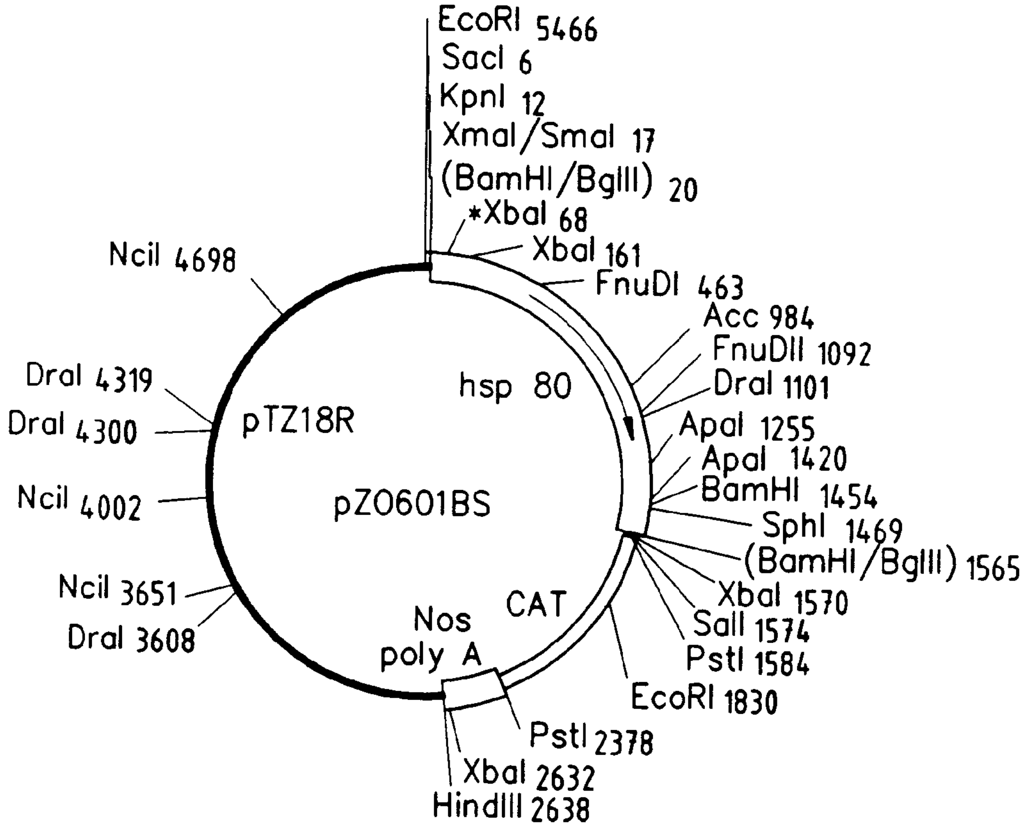
EcoRI 5466
SacI 6
KpnI 12
XmaI/SmaI 17
(BamHI/BglII) 20
*XbaI 68
XbaI 161
FnuDI 463
Acc 984
FnuDII 1092
DraI 1101
ApaI 1255
ApaI 1420
BamHI 1454
SphI 1469
(BamHI/BglII) 1565
XbaI 1570
SalI 1574
PstI 1584
EcoRI 1830
PstI 2378
XbaI 2632
HindIII 2638
NciI 4698
DraI 4319
DraI 4300
NciI 4002
NciI 3651
DraI 3608
hsp 80
pTZ18R
pZO601BS
CAT
Nos
poly A

FIG. 1.2

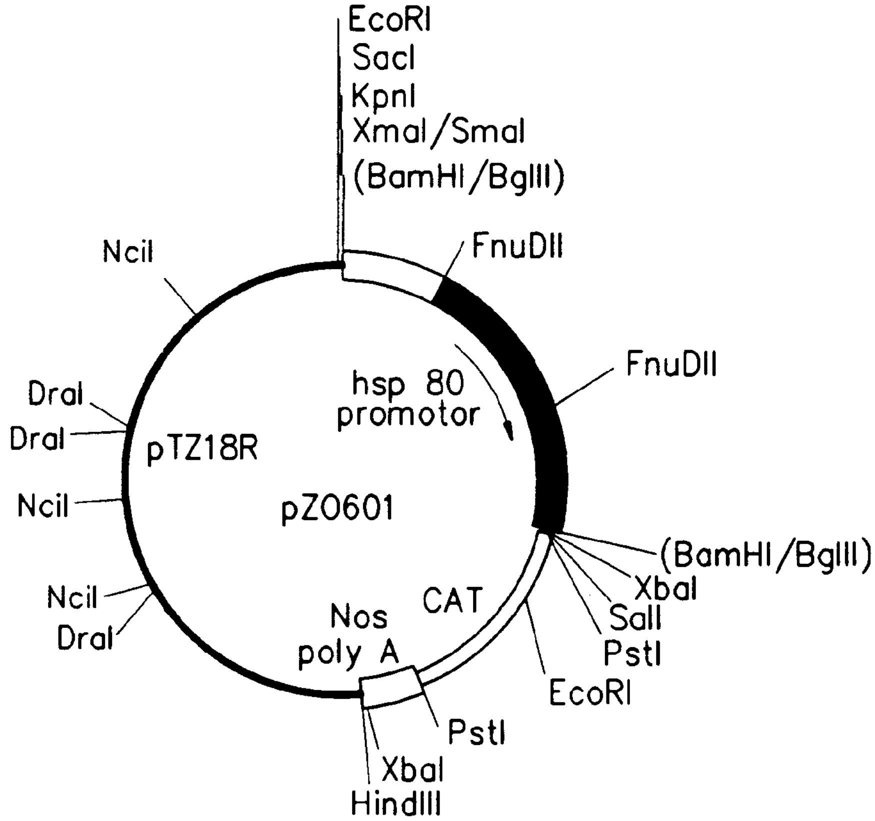
EcoRI
SacI
KpnI
XmaI/SmaI
(BamHI/BglII)
FnuDII
FnuDII
NciI
DraI
DraI
NciI
NciI
DraI
pTZ18R
hsp 80
promotor
pZ0601
CAT
Nos
poly A
(BamHI/BglII)
XbaI
SalI
PstI
EcoRI
PstI
XbaI
HindIII

FIG. 2

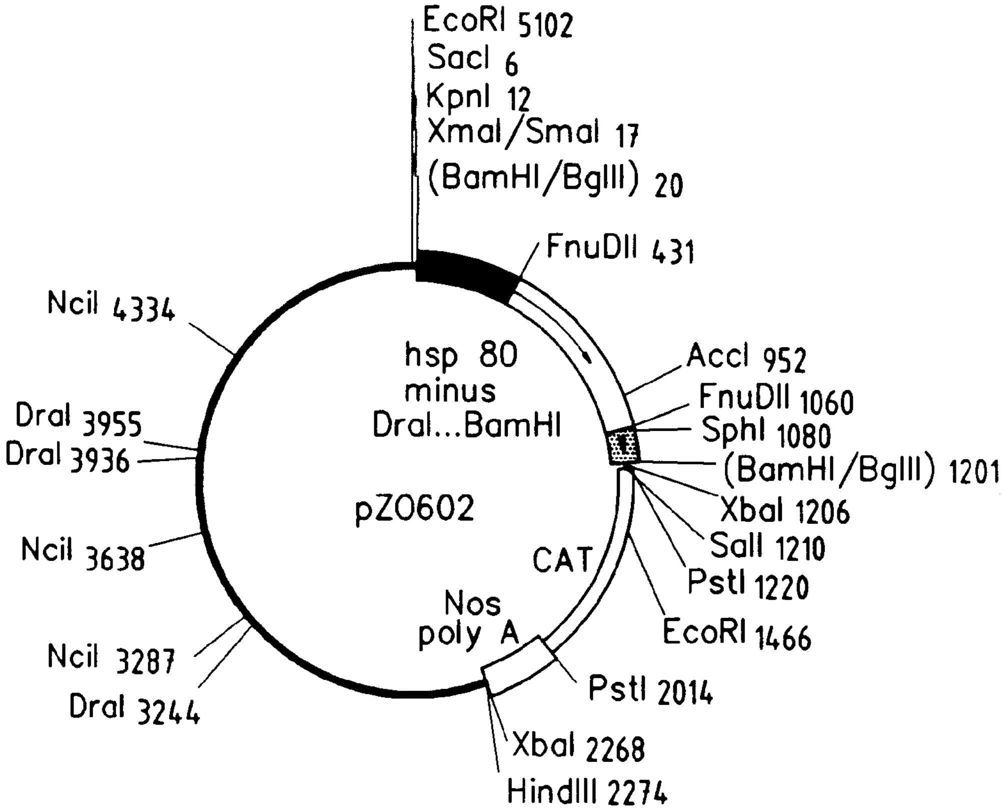
EcoRI 5102
SacI 6
KpnI 12
XmaI/SmaI 17
(BamHI/BglII) 20
FnuDII 431
NciI 4334
hsp 80
minus
DraI...BamHI
AccI 952
FnuDII 1060
SphI 1080
(BamHI/BglII) 1201
XbaI 1206
SalI 1210
PstI 1220
EcoRI 1466
DraI 3955
DraI 3936
pZO602
NciI 3638
CAT
Nos
poly A
NciI 3287
DraI 3244
PstI 2014
XbaI 2268
HindIII 2274

SEQUENCE LISTING

(1) GENERAL INFORMATION:

(i) APPLICANT:
(A) NAME: Sandoz Ltd
(B) STREET: Lichtstrasse 35
(C) CITY: Basel
(D) STATE: BS
(E) COUNTRY: Switzerland
(F) POSTAL CODE (ZIP): CH-4002
(G) TELEPHONE: 061-3242327
(H) TELEFAX: 061-324-7532
(I) TELEX: 965-05055

(A) NAME: Sandoz Patent GMBH
(B) STREET: Humboltstrasse 3
(C) CITY: Lorrach
(E) COUNTRY: Germany
(F) POSTAL CODE (ZIP): D-7850
(G) TELEPHONE: 076 2148014

(A) NAME: Sandoz-Erfindungen Verwaltungsgesellschaft MBH
(B) STREET: Brunner Strasse 59
(C) CITY: Vienna
(E) COUNTRY: Austria
(F) POSTAL CODE (ZIP): A-1235
(G) TELEPHONE: 869361

(ii) TITLE OF INVENTION: Novel Plant Promoter

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 93810003.9

(2) INFORMATION FOR SEQ ID NO: 1:

(i) SEQUENCE CHARACTERISTICS:
(A) LENGTH: 2042 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(xi) SEQUENCE DESCRIPTION: SEQ ID NO: 1:

ATCGATAACC ACGACCACGA CCAAAACCAC GATTGTGACC ACGGCCACGA CCACGCCCAC 60

GATAGGAATA ATTTCCTTTT TCCGGATTTT TTATATCCGT TGCATTTACC TCAGGAAATG 120
CTTTGGTTCC CGTGGGTCGG GCATTGTGGT TTTTAATGAG GAGTTCATTA TTTCTCTCCG 180
CTATTATAAG CGCCACAGCG AGTTCAGAGA ACCTCGTATA CCCACAATTT CTATATTGTT 240
CTTGTAGAAC ATAATGATTT TTGTGGAACG TTTGGTAAGT TTTCTCGAGC ATTTCCGCTT 300
CCGAGACAGG CTTACCGCAA TAATCTAATT GCGCCGCTAT TCTCAATATA GCGGAATTGT 360
ACGTTTCCAC TTTTTCAAAA TCTTGAAATC GTAGATTTTT CCACTCATCG AGTGCATGGG 420
GGAGAGTAAT TTTCTTTTGG TTATCAAACC TCTCCTTCAG AGCTTTCCAA AGATCTAAGG 480
GATCTTTGGT TCTCGCATAA TCATGCGTAA GATTTTCATC TAGATGTCTT CTCAGAAATA 540
TCACGGCCTT AGCCTTTTCA TGAGAGGTTG ATATATTGCC TTCTCTTATT GTTTCGAGTA 600
TTTTCTCGGA ATCTAGATGA AGCTCCATGT TTGTAACCCA TGCCGTGTAG TTTGTCCCAG 660
TTACTTCCAA TGCCGGAAAC TGAAGTTTCT CGATTTTTGC CATTTGTATT TCTAAAGAAC 720
ATAAATAAAA ATTATTAGAA TATTATTCAT ATTAAAAGAA ACCGTTTACA TTGATCATGC 780
AAGCAATTAC AAGGAGAAGC GATGTAAAGA AAAGTAAACC GATATTCATC CTAAATTCTC 840
TTGGAGTAAA TTCTCCAACG GATAAACCAT AAATAGAAAC ACAAATAAAA ATGGCACATA 900
AAAACAAAAG TGCGCGAATC ATCTTTCTTG AAAAAAAAAA TCGGAAGAGA GCGATTTGAA 960
ATTTTTGAGA GAAGATGAAA TATTTTGGAT GATGAAATGG AGTGAAAATG AGTTGTATTT 1020
ATAGATGAAA AACACTGTTC ATAACCGTTG GAGAAAGGGG AAATTTTGAA AAAATTTCTT 1080
TGTGACCGTT GGGGTTAAAT CGAGTGCACT AAAAATCAGT CTGAGAATAT CGTATTAAAC 1140
AGTCAATCAA ATCTATAAAA TTTCATAAAA GTAAAAATTA TGGCAATGAA ATATTTATGT 1200
TATGACAACA AATCATGCGA CGGCTCAGCC GATCAATGCA GAGTAATAAA TAAATTATAC 1260
GGCGGCTCGG CCGACCAATT AATAATAAAC AGAATATAAG GCGGCTCGGC CGACCAATAA 1320
ATAATAAACA GAATATAAGG CGGCTCGGCC GACCATTAAT AAATTAAATT ATTAGTAAAT 1380
AATATAGGCG GTATTCCGGC CATTATAACA TAATATAAAT AATAGTAGAG GCGGTATACC 1440
GACCATTATA ACAGGGTATA AATGATACAA ATAAATTTTA CCGAATCGCA GAGTGATCGT 1500
GCTGATAACG TGTTATGAAA ATAACTGAAA TTTTATTATA TCGCGGGAAT TTAAATAAGG 1560
GCAAAATTTT ATACCCGTAA AAATTATAAC ACTGAAAGAA AGTGTTTATC TGAGAGAGAA 1620
GGGAAGAGTG AAGTGTGTTC TTGAAACGAT CGAACTTGAT CGTATATATA AAGAAAAAAT 1680
CTACTGTGCA AATAGTGCAG CGGGCCCCAC ATCATTTATA ATTTCAACTT ATGCGGCGCT 1740
GTGTTCTCTG ACTTTCATAA CAAAATTATG TTATTTGTTT TAACACAAAA AAGTAGAAAA 1800
TTATAAAGAA GAAGAAAATA ACACATTGAC CAAAAAGAAG TAAATTAGTT ACACCCCAAG 1860
ATTATTGGGC CCAACTTGTC TCAAACTAAC AAGTTAAGCA TAATGGATCT CAGAAGGATC 1920
TAGAAACCCT ATAACGTTTG TGTATATATA CGTAACTTGT CTCTTCACTA CCTCGCATCT 1980

GCTCTCTCTA TTATCGTACC TCCTTGATAA ACCCTAGATC TCCCCGATTC TCAGCAACGA 2040

TG 2042